# EUROPEAN PATENT SPECIFICATION

(11) **EP 1 311 685 B1**
(45) Date of publication and mention of the grant of the patent: **21.03.2007**
(21) Application number: 01980214.9
(22) Date of filing: 19.07.2001
(51) Int. Cl.: C12N 15/31, C12N 1/21, C12N 15/70, C12P 13/08, C12Q 1/68, C12R 1/19, C12R 1/15

(54) **NUCLEOTIDE SEQUENCES WHICH CODE FOR THE CCPA1 GENE**
FÜR DAS CCPA1-GEN KODIERENDE NUKLEOTIDSEQUENZEN
SEQUENCES NUCLEOTIDIQUES CODANT POUR LE GENE ccpA1

(30) Priority: 26.08.2000 DE 10042054; 02.03.2001 DE 10110052
(43) Date of publication of application: 21.05.2003
(73) Proprietor: Degussa GmbH, 40474 Düsseldorf (DE)
(72) Inventor: MÖCKEL, Bettina, 40597 Düsseldorf (DE); KREUTZER, Caroline, 49326 Melle (DE)
(86) International application number: PCT/EP2001/008356
(87) International publication number: WO 2002/018419

(56) References cited:
- EP-A- 1 108 790
- MONEDERO V ET AL: "CATABOLITE REPRESSION IN LACTOBACILLUS CASEI ATCC 393 IS MEDIATED BY CCPA" JOURNAL OF BACTERIOLOGY, WASHINGTON, DC, US, vol. 179, no. 21, November 1997 (1997-11), pages 6657-6664, XP002916657 ISSN: 0021-9193
- EGETER O ET AL: "CATABOLITE REPRESSION MEDIATED BY THE CATABOLITE CONTROL PROTEIN CCPA IN STAPHYLOCOCCUS XYLOSUS" MOLECULAR MICROBIOLOGY, BLACKWELL SCIENTIFIC, OXFORD, GB, vol. 21, no. 4, 1996, pages 739-749, XP002916663 ISSN: 0950-382X
- DATABASE EMBL [Online] Entry Q9RDI2, SEEGER ET AL.: "Probable LacI-family transcriptional regulator" Database accession no. Q9RDI2 XP002199084 & REDENBACH ET AL.: "A set of ordered cosmids and a detailed genetic and physical map for the 8 Mb Streptomyces coelicolor A3(2) chromosome" MOLECULAR MICROBIOLOGY, vol. 21, 1996, pages 77-96,
- C¹CILE JOURLIN-CASTELLI: "CcpC, a novel regulator of the LysR family required for glucose repression of the citB gene in Bacillus subtilis" JOURNAL OF MOLECULAR BIOLOGY, vol. 295, January 2000 (2000-01), pages 865-878, XP002199100

### SEQUENCE PROTOCOL

<110> Degussa AG
<120> New nucleotide sequences which code for the ccpA1 gene
<130> 000059 BT
<140>
   <141>
<160> 4
<170> Patent In Ver. 2.1
<210> 1
   <211> 1600
   <212> DNA
   <213> Corynebacterium glutamicum
<220>
   <221> CDS
   <222> (225)..(1388)
   <223> ccpA1 gene
<400> 1
   tgggttactg cccaggcaat gtttggatag tttttcgggc ttttatcaac agccaataac 60
   agctctttcg cccattgagg tggaggggct gttttttcat gccgtaagga aagtgcaagt 120
   aagtgaaatc aagtggccta gatccattga cacttagact gtgacctagg cttgactttc 180
   gtgggggagt ggggataagt tcatcttaaa cacaatgcaa tcga ttg cat tta cgt  236
                                                    Met His Leu Arg
                                                    1
   tcc tta tcc cac aat agg ggt acc ttc cag aaa gtt ggt gag gag atg   284
   Ser Leu Ser His Asn Arg Gly Thr Phe Gln Lys Val Gly Glu Glu Met
   5                   10                  15                  20
   gct tcc gaa acc tcc agc ccg aag aag cgg gcc acc acg ctc aaa gac   332
   Ala Ser Glu Thr Ser Ser Pro Lys Lys Arg Ala Thr Thr Leu Lys Asp
                   25                  30                  35
   atc gcg caa gca aca cag ctt tca gtc agc acg gtg tcc cgg gca ttg   380
   Ile Ala Gln Ala Thr Gln Leu Ser Val Ser Thr Val Ser Arg Ala Leu
               40                  45                  50
   gcc aac aac gcg agc att ccg gaa tcc aca cgc atc cga gtg gtt gaa   428
   Ala Asn Asn Ala Ser Ile Pro Glu Ser Thr Arg Ile Arg Val Val Glu
           55                  60                  65
   gcc gct caa aag ctg aac tac cgt ccc aat gcc caa gct cgt gca ttg   476
   Ala Ala Gln Lys Leu Asn Tyr Arg Pro Asn Ala Gln Ala Arg Ala Leu
       70                  75                  80
   cgg aag tcg agg aca gac acc atc ggt gtc atc att cca aac att gag   524
   Arg Lys Ser Arg Thr Asp Thr Ile Gly Val Ile Ile Pro Asn Ile Glu
   85                  90                  95                  100
   aac cca tat ttc tcc tca cta gca gca tcg att caa aaa gct gct cgt     572
   Asn Pro Tyr Phe Ser Ser Leu Ala Ala Ser Ile Gln Lys Ala Ala Arg
                   105                 110                 115
   gaa gct ggg gtg tcc acc att ttg tcc aac tct gaa gaa aac cca gag     620
   Glu Ala Gly Val Ser Thr Ile Leu Ser Asn Ser Glu Glu Asn Pro Glu
               120                 125                 130
   ctg ctt ggt cag act ttg gcg atc atg gat gac caa cgc ctc gat gga     668
   Leu Leu Gly Gln Thr Leu Ala Ile Met Asp Asp Gln Arg Leu Asp Gly
           135                 140                 145
   atc atc gtg gtg cca cac att cag tca gag gaa caa gtc act gac ttg     716
   Ile Ile Val Val Pro His Ile Gln Ser Glu Glu Gln Val Thr Asp Leu
       150                 155                 160
   gtt aac agg gga gtg cca gta gtg ctg gca gac cgt agt ttt gtt aac     764
   Val Asn Arg Gly Val Pro Val Val Leu Ala Asp Arg Ser Phe Val Asn
   165                 170                 175                 180
   tcg tct att cct tcg gtt acc tca gat cca gtt ccg ggc atg act gaa     812
   Ser Ser Ile Pro Ser Val Thr Ser Asp Pro Val Pro Gly Met Thr Glu
                   185                 190                 195
   gct gtg gac tta ctc ctg gca gct gac gtg caa ttg ggc tac ctt gcc     860
   Ala Val Asp Leu Leu Leu Ala Ala Asp Val Gln Leu Gly Tyr Leu Ala
               200                 205                 210
   ggc ccg cag gat act tcc act ggt cag ctg cgt ctt aac act ttt gaa     908
   Gly Pro Gln Asp Thr Ser Thr Gly Gln Leu Arg Leu Asn Thr Phe Glu
           215                 220                 225
   aga cta tgc gtg gac cgc ggc atc gtc gga gca tct gtc tat tac ggt     956
   Arg Leu Cys Val Asp Arg Gly Ile Val Gly Ala Ser Val Tyr Tyr Gly
       230                 235                 240
   ggc tac cgc caa gaa tct gga tat gac ggc atc aag gtg ctg atc aag     1004
   Gly Tyr Arg Gln Glu Ser Gly Tyr Asp Gly Ile Lys Val Leu Ile Lys
   245                 250                 255                 260
   cag gga gcc aat gcg att atc gct ggt gac tcc atg atg acc atc ggt     1052
   Gln Gly Ala Asn Ala Ile Ile Ala Gly Asp Ser Met Met Thr Ile Gly
                   265                 270                 275
   gcg ttg ttg gct ctt cat gag atg aat ttg aag atc ggt gag gat gtg     1100
   Ala Leu Leu Ala Leu His Glu Met Asn Leu Lys Ile Gly Glu Asp Val
               280                 285                 290
   cag ctc att ggg ttt gat aac aac cca att ttc cgg ctg cag aat cca     1148
   Gln Leu Ile Gly Phe Asp Asn Asn Pro Ile Phe Arg Leu Gln Asn Pro
           295                 300                 305
   ccg ctg agc atc att gac cag cac gta caa gag atc ggt aag cgt gcg     1196
   Pro Leu Ser Ile Ile Asp Gln His Val Gln Glu Ile Gly Lys Arg Ala
       310                 315                 320
   ttt gag att ctg cag aag ctg atc aat ggg gac acc gcg caa aaa tct     1244
   Phe Glu Ile Leu Gln Lys Leu Ile Asn Gly Asp Thr Ala Gln Lys Ser
   325                 330                 335                 340
   gtg gtg att cca acg cag ctc agc atc aat gga tca acg gcg gtt tcc   1292
   Val Val Ile Pro Thr Gln Leu Ser Ile Asn Gly Ser Thr Ala Val Ser
                   345                 350                 355
   caa aag gcg gcc gca aag gca gca aaa gca gcc caa aaa gca gcc gcg   1340
   Gln Lys Ala Ala Ala Lys Ala Ala Lys Ala Ala Gln Lys Ala Ala Ala
               360                 365                 370
   aaa gcc gca cag aac acg caa cac gag gtg agc cta gat ggt gaa ctc   1388
   Lys Ala Ala Gln Asn Thr Gln His Glu Val Ser Leu Asp Gly Glu Leu
           375                 380                 385
   tgaacaagcg cttcatcagc atgatcctgc accaatcctt cagttggata aagtctccaa 1448
   gtcgtttggc ccagtcaacg tcattaatca agtgagcatc gatgttcgcc ctggcagggt 1508
   gcttgcgctg ttgggtgaaa atggtgcggg taaatctacg ctgatcaaga tgatgtcggg 1568
   tgtgtatcag cctgatggcg ggcagatttt gg                               1600
<210> 2
   <211> 388
   <212> PRT
   <213> Corynebacterium glutamicum
<400> 2
   Met His Leu Arg Ser Leu Ser His Asn Arg Gly Thr Phe Gln Lys Val
    1               5                  10                  15
   Gly Glu Glu Met Ala Ser Glu Thr Ser Ser Pro Lys Lys Arg Ala Thr
               20                  25                  30
   Thr Leu Lys Asp Ile Ala Gln Ala Thr Gln Leu Ser Val Ser Thr Val
           35                  40                  45
   Ser Arg Ala Leu Ala Asn Asn Ala Ser Ile Pro Glu Ser Thr Arg Ile
       50                  55                  60
   Arg Val Val Glu Ala Ala Gln Lys Leu Asn Tyr Arg Pro Asn Ala Gln
   65                  70                  75                  80
   Ala Arg Ala Leu Arg Lys Ser Arg Thr Asp Thr Ile Gly Val Ile Ile
                   85                  90                  95
   Pro Asn Ile Glu Asn Pro Tyr Phe Ser Ser Leu Ala Ala Ser Ile Gln
               100                 105                 110
   Lys Ala Ala Arg Glu Ala Gly Val Ser Thr Ile Leu Ser Asn Ser Glu
           115                 120                 125
   Glu Asn Pro Glu Leu Leu Gly Gln Thr Leu Ala Ile Met Asp Asp Gln
       130                 135                 140
   Arg Leu Asp Gly Ile Ile Val Val Pro His Ile Gln Ser Glu Glu Gln
   145                 150                 155                 160
   Val Thr Asp Leu Val Asn Arg Gly Val Pro Val Val Leu Ala Asp Arg
                   165                 170                 175
   Ser Phe Val Asn Ser Ser Ile Pro Ser Val Thr Ser Asp Pro Val Pro
               180                 185                 190
   Gly Met Thr Glu Ala Val Asp Leu Leu Leu Ala Ala Asp Val Gln Leu
           195                 200                 205
   Gly Tyr Leu Ala Gly Pro Gln Asp Thr Ser Thr Gly Gln Leu Arg Leu
       210                 215                 220
   Asn Thr Phe Glu Arg Leu Cys Val Asp Arg Gly Ile Val Gly Ala Ser
   225                 230                 235                 240
   Val Tyr Tyr Gly Gly Tyr Arg Gln Glu Ser Gly Tyr Asp Gly Ile Lys
                   245                 250                 255
   Val Leu Ile Lys Gln Gly Ala Asn Ala Ile Ile Ala Gly Asp Ser Met
               260                 265                 270
   Met Thr Ile Gly Ala Leu Leu Ala Leu His Glu Met Asn Leu Lys Ile
           275                 280                 285
   Gly Glu Asp Val Gln Leu Ile Gly Phe Asp Asn Asn Pro Ile Phe Arg
       290                 295                 300
   Leu Gln Asn Pro Pro Leu Ser Ile Ile Asp Gln His Val Gln Glu Ile
   305                 310                 315                 320
   Gly Lys Arg Ala Phe Glu Ile Leu Gln Lys Leu Ile Asn Gly Asp Thr
                   325                 330                 335
   Ala Gln Lys Ser Val Val Ile Pro Thr Gln Leu Ser Ile Asn Gly Ser
               340                 345                 350
   Thr Ala Val Ser Gln Lys Ala Ala Ala Lys Ala Ala Lys Ala Ala Gln
           355                 360                 365
   Lys Ala Ala Ala Lys Ala Ala Gln Asn Thr Gln His Glu Val Ser Leu
       370                 375                 380
   Asp Gly Glu Leu
   385
<210> 3
   <211> 20
   <212> DNA
   <213> Corynebacterium glutamicum
<220>
   <223> Primer ccpA1intA
<400> 3
   agagctgctt ggtcagactt 20
<210> 4
   <211> 20
   <212> DNA
   <213> Corynebacterium glutamicum
<220>
   <223> Primer ccpAlintB
<400> 4
   atccagattc ttggcggtag 20

## Description

The invention provides a process for the fermentative preparation of L-lysine, by elimination of the ccpA1 gene. The ccpA1 gene codes for the CcpA1 protein, which is a catabolite control protein A.

### Prior Art

L-Amino acids, in particular L-lysine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and which produce amino acids are obtained in this manner.

Methods of the recombinant DNA technique have also been employed for some years for improving the strain of Corynebacterium strains which produce L-amino acid.

In Journal of Bacteriology, 1997, age 6657-6664 a Lactobacillus casei catabolite regulator protein CcpA is disclosed, which has however no relevant sequence identity to SEQ ID NO: 2 of the resent description. This document does not give any hint concerning a possible influence of a CcpA-protein on the L-Lysine production.

### Object of the Invention

The inventors had the object of providing new measures for improved fermentative preparation of L-lysine.

### Description of the Invention

If L-lysine or lysine are mentioned in the following, this also means the salts, such as e.g. lysine monohydrochloride or lysine sulfate.

The description provides an isolated polynucleotide from coryneform bacteria, comprising a polynucleotide sequence which codes for the ccpA1 gene, chosen from the group consisting of
a) polynucleotide which is identical to the extent of at least 70% to a polynucleotide which codes for a polypeptide which comprises the amino acid sequence of SEQ ID No. 2,
b) polynucleotide which codes for a polypeptide which comprises an amino acid sequence which is identical to the extent of at least 70% to the amino acid sequence of SEQ ID No. 2,
c) polynucleotide which is complementary to the polynucleotides of a) or b), and
d) polynucleotide comprising at least 15 successive nucleotides of the polynucleotide sequence of a), b) or c),
the polypeptide having the activity of the catabolite control protein CcpA1.

The description also provides the abovementioned polynucleotide, this preferably being a DNA which is capable of replication, comprising:
(i) the nucleotide sequence shown in SEQ ID No.1 or
(ii) at least one sequence which corresponds to sequence (i) within the range of the degeneration of the genetic code, or
(iii) at least one sequence which hybridizes with the sequences complementary to sequences (i) or (ii), and optionally
(iv) sense mutations of neutral function in (i).

The description also provides:
a DNA which is capable of replication and comprises the nucleotide sequence as shown in SEQ ID No.1; a polynucleotide which codes for a polypeptide which comprises the amino acid sequence as shown in SEQ ID No. 2;

The invention provides:
a vector containing parts of the polynucleotide according to the description, but at least 15 successive nucleotides of the sequence claimed;
and coryneform bacteria in which the ccpA1 gene is eliminated, in particular by an insertion or deletion.

The description also provides polynucleotides which substantially comprise a polynucleotide sequence, which are obtainable by screening by means of hybridization of a corresponding gene library of a coryneform bacterium, which comprises the complete gene or parts thereof, with a probe which comprises the sequence of the polynucleotide according to the invention according to SEQ ID No.1 or a fragment thereof, and isolation of the polynucleotide sequence mentioned.

Polynucleotide sequences according to the description are suitable as hybridization probes for RNA, cDNA and DNA, in order to isolate, in the full length, nucleic acids or polynucleotides or genes which code for the CcpA1 protein or to isolate those nucleic acids or polynucleotides or genes which have a high similarity with the sequence of the ccpA1 gene.

Polynucleotide sequences according to the description are furthermore suitable as primers with the aid of which DNA of genes which code for the CcpA1 protein can be prepared with the polymerase chain reaction (PCR).

Such oligonucleotides which serve as probes or primers comprise at least 30, preferably at least 20, very particularly preferably at least 15 successive nucleotides. Oligonucleotides which have a length of at least 40 or 50 nucleotides are also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

The polynucleotides according to the description include a polynucleotide according to SEQ ID No. 1 or a fragment prepared therefrom and also those which are at least 70%, preferably at least 80% and in particular at least 90% to 95% identical to the polynucleotide according to SEQ ID No. 1 or a fragment prepared therefrom.

"Polypeptides" are understood as meaning peptides or proteins which comprise two or more amino acids bonded via peptide bonds.

The polypeptides according to the description include a polypeptide according to SEQ ID No. 2, in particular those with the biological activity of the CcpA1 protein, and also those which are at least 70%, preferably at least 80% and in particular at least 90% to 95% identical to the polypeptide according to SEQ ID No. 2 and have the activity mentioned.

The invention moreover provides a process for the fermentative preparation of L-lysine, using coryneform bacteria which in particular already produce L-Lysine, and in which the nucleotide sequences which code for the ccpA1 gene are eliminated.

The term "attenuation" in this connection describes the elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

The term "enhancement" in this connection describes the increase in the intracellular activity of one or more enzymes in a microorganism which are coded by the corresponding DNA, for example by increasing the number of copies of the gene or genes, using a potent promoter or using a gene which codes for a corresponding enzyme having a high activity, and optionally combining these measures.

The microorganisms which the present invention provides can prepare L-lysine, from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-Lysine.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
or L-Lysine-producing mutants or strains prepared therefrom, such as, for example, the L-lysine-producing strains
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum DM58-1
Corynebacterium glutamicum DG52-5
Corynebacterium glutamicum DSM 5715 and
Corynebacterium glutamicum DSM 12866

The inventors have succeeded in isolating the ccpA1 gene of C. glutamicum which codes for the CcpA1 protein, which is a catabolite control protein A.

To isolate the ccpA1 gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einfuhrung in die Gentechnologie [Genes and Clones, An Introduction to Genetic Engineering] (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495-508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575). Börmann et al. (Molecular Microbiology 6(3), 317-326)) (1992)) in turn describe a gene library of C. glutamicum ATCC13032 using the cosmid pHC79 (Hohn and Collins, 1980, Gene 11, 291-298).

To prepare a gene library of C. glutamicum in E. coli it is also possible to use plasmids such as pBR322 (Bolivar, 1979, Life Sciences, 25, 807-818) or pUC9 (Vieira et al., 1982, Gene, 19:259-268). Suitable hosts are, in particular, those E. coli strains which are restriction- and recombination-defective, such as, for example, the strain DH5α (Jeffrey H. Miller: "A Short Course in Bacterial Genetics, A Laboratory Manual and Handbook for Escherichia coli and Related Bacteria", Cold Spring Harbour Laboratory Press, 1992).

The long DNA fragments cloned with the aid of cosmids or other λ-vectors can then be subcloned in turn into the usual vectors suitable for DNA sequencing.

Methods of DNA sequencing are described, inter alia, by Sanger et al. (Proceedings of the National Academy of Sciences of the United States of America USA, 74:5463-5467, 1977).

The resulting DNA sequences can then be investigated with known algorithms or sequence analysis programs, such as e.g. that of Staden (Nucleic Acids Research 14, 217-232 (1986)), that of Marck (Nucleic Acids Research 16, 1829-1836 (1988)) or the GCG program of Butler (Methods of Biochemical Analysis 39, 74-97 (1998)).

The DNA sequence of C. glutamicum which codes for the ccpA1 gene and which, as SEQ ID No. 1, is a constituent of the present description has been found in this manner. The amino acid sequence of the corresponding protein has furthermore been derived from the present DNA sequence by the methods described above. The resulting amino acid sequence of the ccpA1 gene product is shown in SEQ ID No. 2.

Coding DNA sequences which result from SEQ ID No. 1 by the degeneracy of the genetic code are also a constituent of the description. In the same way, DNA sequences which hybridize with SEQ ID No. 1 or parts of SEQ ID No. 1 are a constituent of the description. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the description.

Finally, DNA sequences which are prepared by the polymerase chain reaction (PCR) using primers which result from SEQ ID No. 1 are a constituent of the description. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology 41: 255-260 (1991)). The hybridization takes place under stringent conditions, that is to say only hybrids in which the probe and target sequence, i.e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50 - 68°C, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and subsequently 0.5x SSC (The DIG System User's Guide for Filter Hybridisation, Boehringer Mannheim, Mannheim, Germany, 1995) a temperature of approx. 50 - 68°C being established. It is optionally possible to lower the salt concentration to 0.1x SSC. Polynucleotide fragments which are, for example, at least 70% or at least 80% or at least 90% to 95% identical to the sequence of the probe employed can be isolated by increasing the hybridization temperature stepwise in steps of approx. 1 - 2°C. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

In the work on the present invention, it has been found that coryneform bacteria produce L-lysine, in an improved manner after elimination of the ccpA1 gene.

To achieve an elimination, either the expression of the ccpA1 gene or the catalytic properties of the enzyme protein can be eliminated. The two measures can optionally be combined.

The reduction in gene expression can take place by suitable culturing or by genetic modification (mutation) of the signal structures of gene expression. Signal structures of gene expression are, for example, repressor genes, activator genes, operators, promoters, attenuators, ribosome binding sites, the start codon and terminators. The expert can find information on this e.g. in the patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pátek et al. (Microbiology 142: 1297 (1996)), Vasicova et al. (Journal of Bacteriology 181: 6188 (1999)) and in known textbooks of genetics and molecular biology, such as e.g. the textbook by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH.Verlagsgesellschaft, Weinheim, Germany, 1990).

Mutations which lead to a change or reduction in the catalytic properties of enzyme proteins are known from the prior art; examples which may be mentioned are the works by Qiu and Goodman (Journal of Biological Chemistry 272: 8611-8617 (1997)), Sugimoto et al. (Bioscience Biotechnology and Biochemistry 61: 1760-1762 (1997)) and Mockel ("Die. Threonindehydratase aus Corynebacterium glutamicum: Aufhebung der allosterischen Regulation und Struktur des Enzyms [Threonine dehydratase from Corynebacterium glutamicum: Cancelling the allosteric regulation and structure of the enzyme]", Reports from the Jülich Research Centre, Jül-2906, ISSN09442952, Jülich, Germany, 1994). Summarizing descriptions can be found in known textbooks of genetics and molecular biology, such as e.g. that by Hagemann ("Allgemeine Genetik [General Genetics]", Gustav Fischer Verlag, Stuttgart, 1986).

Possible mutations are transitions, transversions, insertions.and deletions. Depending on the effect of the amino acid exchange on the enzyme activity, "missense mutations" or "nonsense mutations" are referred to. Insertions or deletions of at least one base pair (bp) in a gene lead to frame shift mutations, as a consequence of which incorrect amino acids are incorporated or translation is interrupted prematurely. Deletions of several codons typically lead to a complete loss of the enzyme activity. Instructions on generation of such mutations are prior art and can be found in known textbooks of genetics and molecular biology, such as e.g. the textbook by Knippers ("Molekulare Genetik [Molecular Genetics]", 6th edition, Georg Thieme Verlag, Stuttgart, Germany, 1995), that by Winnacker ("Gene und Klone [Genes and Clones]", VCH Verlagsgesellschaft, Weinheim, Germany, 1990) or that by Hagemann ("Allgemeine Genetik [General Genetics]", Gustav Fischer Verlag, Stuttgart, 1986).

A common method of mutating genes of C. glutamicum is the method of "gene disruption" and "gene replacement" described by Schwarzer and Pühler (Bio/Technology 9, 84-87 (1991)).

In the method of gene disruption a central part of the coding region of the gene of interest is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pK18mobsacB or pK19mobsacB (Jäger et al., Journal of Bacteriology 174: 5462-65 .(1992)), pGEM-T (Promega corporation, Madison, WI, USA), pCR2.1-TOPO (Shuman (1994). Journal of Biological Chemistry 269:32678-84; US Patent 5,487,993), pCR®Blunt (Invitrogen, Groningen, The Netherlands; Bernard et al., Journal of Molecular Biology, 234: 534-541 (1993)) or pEM1 (Schrumpf et al, 1991, Journal of Bacteriology 173:4510-4516). The plasmid vector which contains the central part of the coding region of the gene is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross-over" event, the coding region of the gene in question is interrupted by the vector sequence and two incomplete alleles are obtained, one lacking the 3' end and one lacking the 5' end. This method has been used, for example, by Fitzpatrick et al. (Applied Microbiology and Biotechnology 42, 575-580 (1994)) to eliminate the recA gene of C. glutamicum.

In the method of "gene replacement", a mutation, such as e.g. a deletion, insertion or base exchange, is established in vitro in the gene of interest. The allele prepared is in turn cloned in a vector which is not replicative for C. glutamicum and this is then transferred into the desired host of C. glutamicum by transformation or conjugation. After homologous recombination by means of a first "cross-over" event which effects integration and a suitable second "cross-over" event which effects excision in the target gene or in the target sequence, the incorporation of the mutation or of the allele is achieved. This method was used, for example, by Peters-Wendisch et al. (Microbiology 144, 915 - 927 (1998)) to eliminate the pyc gene of C. glutamicum by a deletion.

A deletion, insertion or a base exchange can be incorporated into the ccpA1 gene in this manner.

In addition, it may be advantageous for the production of L-lysine, to enhance, in particular to over-express, one or more enzymes of the particular biosynthesis pathway, of glycolysis, of anaplerosis, of the citric acid cycle, of the pentose phosphate cycle or of amino acid export and optionally regulatory proteins, in addition to elimination of the ccpA1 gene.

The term "enhancement" in this connection describes the increase in the intracellular activity of one or more enzymes (proteins) in a microorganism which are coded by the corresponding DNA, for example by increasing the number of copies of the gene or genes, using a potent promoter or using a gene or allele which codes for a corresponding enzyme (protein) having a high activity, and optionally combining these measures.

Thus, for the preparation of L-lysine, at the same time one or more of the genes chosen from the group consisting of
- the dapA gene which codes for dihydrodipicolinate synthase (EP-B 0 197 335),
- the eno gene which codes for enolase (DE: 19947791.4),
- the zwf gene which codes for the zwf gene product (JP-A-09224661),
- at the same time the dapD gene which codes for tetradihydrodipicolinate succinylase (Wehrmann et al., Journal of Bacteriology 180, 3159-3165 (1998)),
- at the same time the dapE gene which codes for succinyl diaminopimelate desuccinylase (Wehrmann et al., Journal of Bacteriology 177: 5991-5993 (1995)),
- at the same time the gap gene which codes for glyceraldehyde 3-phosphate dehydrogenase (Eikmanns (1992). Journal of Bacteriology 174:6076-6086),
- the pyc gene which codes for pyruvate carboxylase (Peters-Wendisch et al. (Microbiology 144, 915 - 927 (1998))
- at the same time the mqo gene which codes for malate:quinone oxidoreductase (Molenaar et al., European Journal of Biochemistry 254, 395-403 (1998)),
- the lysC gene which codes for a feed back resistant aspartate kinase (Kalinowski et al. (1990), Molecular and General Genetics 224, 317-324; Accession No.P26512; EP-B-0387527; EP-A-0699759; WO 00/63388),
- the zwa1 gene which codes for the Zwa1 protein (DE: 19959328.0, DSM 13115),
- the lysE gene which codes for lysine export (DE-A-195 48 222)
can be enhanced, in particular over-expressed.

It may furthermore be advantageous for the production of L-lysine, in addition to the elimination of the ccpA1 gene, at the same time for one or more of the genes chosen from the group consisting of
- the pck gene which codes for phosphoenol pyruvate carboxykinase (DE 199 50 409.1, DSM 13047),
- the pgi gene which codes for glucose 6-phosphate isomerase (US 09/396,478, DSM 12969),
- the zwa2 gene which codes for the Zwa2 protein (DE: 19959327.2, DSM 13113),
- the poxB gene which codes for pyruvate oxidase DE:1995 1975.7, DSM 13114)
to be eliminated.

In addition to elimination of the ccpA1 gene it may furthermore be advantageous for the production of L-lysine, to eliminate undesirable side reactions, (Nakayama: "Breeding of Amino Acid Producing Microorganisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of L-lysine. A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981). Sugars and carbohydrates, such as e.g. glucose, sucrose, lactose, fructose, maltose, molasses, starch and cellulose, oils and fats, such as, for example, soya oil, sunflower oil, groundnut oil and coconut fat, fatty acids, such as, for example, palmitic acid, stearic acid and linoleic acid, alcohols, such as, for example, glycerol and ethanol, and organic acids, such as, for example, acetic acid, can be used as the source of carbon. These substance can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as, for example, magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as, for example, fatty acid polyglycol esters, can be employed to control the development of foam. Suitable substances having a selective action, such as, for example, antibiotics, can be added to the medium to maintain the stability of plasmids. To maintain aerobic conditions, oxygen or oxygen-containing gas mixtures, such as, for example, air, are introduced into the culture. The temperature of the culture is usually 20°C to 45°C, and preferably 25°C to 40°C. Culturing is continued until a maximum of the desired product has formed. This target is usually reached within 10 hours to 160 hours.

Methods for the determination of L-amino acids are known from the prior art. The analysis can thus be carried out, for example, as described by Spackman et al. (Analytical Chemistry, 30, (1958), 1190) by anion exchange chromatography with subsequent ninhydrin derivatization, or it can be carried out by reversed phase HPLC, for example as described by Lindroth et al. (Analytical Chemistry (1979) 51: 1167-1174).

The process according to the invention is used for the fermentative preparation of L-lysine.

The following microorganism was deposited on 22.08.2000 as a pure culture at the Deutsche Sammlung für Mikroorganismen und Zellkulturen (DSMZ = German Collection of Microorganisms and Cell Cultures, Braunschweig, Germany) in accordance with the Budapest Treaty:
- Escherichia coli strain Top10F/pCR2.1ccpA1int DSM 13673.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual, 1989, Cold Spring Harbour Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from C. glutamicum ATCC 13032

Chromosomal DNA from C. glutamicum ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCos1 (Wahl et al. (1987), Proceedings of the National Academy of Sciences, USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Res. 16:1563-1575) the cells were taken up in 10 mM MgSO₄ and mixed with an aliquot of the phage suspension. The infection and titering of the cosmid library were carried out as described by Sambrook et al. (1989, Molecular Cloning: A Laboratory Manual, Cold Spring Harbor), the cells being plated out on LB agar (Lennox, 1955, Virology, 1:190) + 100 µg/ml ampicillin. After incubation overnight at 37°C, recombinant individual clones were selected.

### Example 2

### Isolation and sequencing of the ccpA1 gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, The-Netherlands, Product Description Zero Background Cloning Kit, Product No. K2500-01) was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5αMCR (Grant, 1990, Proceedings of the National Academy of Sciences, U.S.A., 87:4645-4649) and plated out on LB agar (Lennox, 1955, Virology, 1:190) with 50 µg/ml zeocin.

The plasmid preparation of the recombinant clones was carried out with Biorobot 9600 (Product No. 900200, Qiagen, Hilden, Germany). The sequencing was carried out by the dideoxy chain termination method of Sanger et al. (1977, Proceedings of the National Academies of Sciences, U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analyses were prepared with the XNIP program (Staden, 1986, Nucleic Acids Research, 14:217-231). Further analyses were carried out with the "BLAST search program" (Altschul et al., 1997, Nucleic Acids Research, 25:33893402) against the non-redundant databank of the "National Center for Biotechnology Information" (NCBI, Bethesda, MD, USA).

The resulting nucleotide sequence is shown in SEQ ID No. 1. Analysis of the nucleotide sequence showed an open reading frame of 1167 bp, which was called the ccpA1 gene. The ccpA1 gene codes for a polypeptide of 388 amino acids. '

### Example 3

### Preparation of an integration vector for integration mutagenesis of the ccpA1 gene

From the strain ATCC 13032, chromosomal DNA was isolated by the method of Eikmanns et al. (Microbiology 140: 1817 - 1828 (1994)). On the basis of the sequence of the ccpA1 gene known for C. glutamicum from example 2, the following oligonucleotides were chosen for the polymerase chain reaction (see SEQ ID No. 3 and SEQ ID No. 4):
ccpA1intA:
   5'AGA GCT GCT TGG TCA GAC TT 3'
ccpA1intB:
   5'ATC CAG ATT CTT GGC GGT AG 3'

The primers shown were synthesized by MWG Biotech (Ebersberg, Germany) and the PCR reaction was carried out by the standard PCR method of Innis et al. (PCR protocols. A guide to methods and applications, 1990, Academic Press) with Pwo-Polymerase from Boehringer. With the aid of the polymerase chain reaction, an internal fragment of the ccpA1 gene 362 bp in size was isolated.

The amplified DNA fragment was ligated with the TOPO TA Cloning Kit from Invitrogen Corporation (Carlsbad, CA, USA; Catalogue Number K4500-01) in the vector pCR2.1-TOPO (Mead at al. (1991), Bio/Technology 9:657-663).

The E. coli strain TOP10F was then transformed with the ligation batch (Hanahan, In: DNA cloning. A practical approach. Vol. I, IRL-Press, Oxford, Washington DC, USA 1985). Selection for plasmid-carrying cells was made by plating out the transformation batch on LB agar (Sambrook et al., Molecular Cloning: A Laboratory Manual. 2^{nd} Ed., Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCR2.1ccpA1int.

### Example 4

### Integration mutagenesis of the ccpA1 gene in the lysine producer DSM 5715

The vector pCR2.1ccpAlint mentioned in example 3 was electroporated by the electroporation method of Tauch et al. (FEMS Microbiological Letters, 123:343-347 (1994)) in C. glutamicum DSM 5715. The strain DSM 5715 is an AEC-resistant lysine producer. The vector pCR2.1ccpA1int cannot replicate independently in DSM 5715 and is retained in the cell only if it has integrated into the chromosome of DSM 5715. Selection of clones with pCR2.1ccpAlint integrated into the chromosome was carried out by plating out the electroporation batch on LB agar (Sambrook et al., Molecular Cloning: A Laboratory Manual. 2^{nd} Ed., Cold Spring Harbor Laboratory Press, Cold Spring Harbor, N.Y.), which had been supplemented with 15 mg/l kanamycin.

For detection of the integration, the ccpA1int fragment was labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant was isolated by the method of Eikmanns et al. (Microbiology 140: 1817 - 1828 (1994)) and in each case cleaved with the restriction enzymes PstI, SacI and HindIII. The fragments formed were separated by agarose gel electrophoresis and hybridized at 68°C with the Dig hybridization kit from Boehringer. The plasmid pCR2.1ccpAlint mentioned in example 3 had been inserted into the chromosome of DSM 5715 within the chromosomal ccpA1 gene. The strain was called DSM 5715::pCR2.1ccpA1int.

### Example 5

### Preparation of L-lysine

The C. glutamicum strain DSM 5715::pCR2.1ccpA1int obtained in example 4 was cultured in a nutrient medium suitable for the production of L-lysine and the L-lysine content in the culture supernatant was determined.

For this, the strain was first incubated on an agar plate with the corresponding antibiotic (brain-heart agar with kanamycin (25 mg/l) for 24 hours at 33°C. Starting from this agar plate culture, a preculture was seeded (10 ml medium in a 100 ml conical flask). The complete medium CgIII was used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Kanamycin (25 mg/l) was added to this. The preculture was incubated for 24 hours at 33°C at 240 rpm on a shaking machine. A main culture was seeded from this preculture such that the initial OD (660 nm) of the main culture was 0.1 OD. Medium MM was used for the main culture.

| | |
|---|---|
| Medium MM | |
| CSL (corn steep liquor) | 5 g/l |
| MOPS (morpholinopropanesulfonic acid) | 20 g/l |
| Glucose (autoclaved separately) | 50g/l |
| Salts: | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| Leucine (sterile-filtered) | 0.1 g/l |
| CaCO₃ | 25 g/l |

The CSL, MOPS and the salt solution are brought to pH 7 with aqueous ammonia and autoclaved. The sterile substrate and vitamin solutions are then added, as well as the CaCO₃ autoclaved in the dry state.

Culturing is carried out in a 10 ml volume in a 100 ml conical flask with baffles. Kanamycin (25 mg/1) was added. Culturing was carried out at 33°C and 80% atmospheric humidity. After 72 hours, the OD was determined at a measurement wavelength of 660 nm with a Biomek 1000 (Beckmann Instruments GmbH, Munich). The amount of L-lysine formed was determined with an amino acid analyzer from Eppendorf-BioTronik (Hamburg, Germany) by ion exchange chromatography and post-column derivatization with ninhydrin detection.

The result of the experiment is shown in table 1.

**Table 1**

| Strain | OD (660 nm) | Lysine HCl g/l |
|---|---|---|
| DSM 5715 | 7.5 | 13.01 |
| DSM 5715::pCR2.1ccpA1int | 7.7 | 14.24 |

### Brief Description of the Figure:

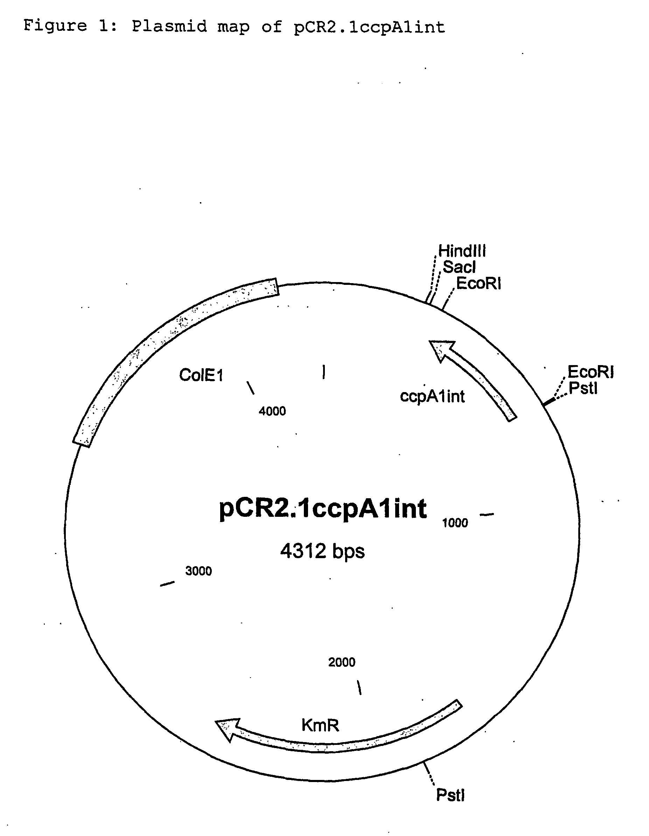

Figure 1: Map of the plasmid pCR2.1ccpA1int.

The abbreviations and designations used have the following meaning.
KmR: Kanamycin resistance gene
EcoRI: Cleavage site of the restriction enzyme EcoRI
HindIII: cleavage site of the restriction enzyme HindIII
PstI: Cleavage site of the restriction enzyme PstI
SacI: Cleavage site of the restriction enzyme SacI
ccpA1int: Internal fragment of the ccpA1 gene
ColE1 ori: Replication origin of the plasmid ColE1

## Claims

1. An isolated coryneform bacterium in which the expression of a polynucleotide encoding a polypeptide having an amino acid sequence which is at least 90 % identical to the amino acid sequence set out in SEQ ID NO:2 is eliminated, wherein the polypeptide has the function of a catabolite control protein.

2. The bacterium of claim 1, wherein said bacterium is a bacterium of the species Corynebacterium glutamicum.

3. The bacterium of claim 1, wherein said polypeptide has the amino acid sequence set out in SEQ ID NO:2.

4. The bacterium of claim 1, wherein said polynucleotide has the nucleotide sequence of nucleotides 225 to 1388 of SEQ ID NO:1.

5. The bacterium of claim 4, wherein said polynucleotide has the nucleotide sequence of SEQ ID NO:1.

6. The bacterium of claim 1 or 2, wherein said bacterium has the ability to accumulate L-lysine in its cells or in a medium.

7. A process for the preparation of L-lysine comprising:
fermentation of the bacteria according to one of the claims 1 to 6 which produce L-lysine.

8. A process for the preparation of L-lysine comprising
8.1 fermentation of the bacteria of claims 1 to 6,
8.2 concentration of the L-lysine, in the medium or in the cells of the bacteria, and
8.3 isolation of the L-lysine.

9. A process according to claim 7 or 8, wherein bacteria in which further genes of the biosynthesis pathway of L-lysine are additionally enhanced are employed.

10. A process according to one or more of the claims 7-9, wherein bacteria in which the metabolic pathways which reduce the formation of L-lysine are eliminated are employed.

11. A process according to claim 9, wherein bacteria are fermented in which at the same time one or more of the genes chosen from the group consisting of
11.1 the dapA gene which codes for dihydrodipicolinate synthase,
11.2 the eno gene which codes for enolase,
11.3 the zwf gene which codes for the zwf gene product,
11.4 the pyc gene which codes for pyruvate carboxylase,
11.5 the lysE gene which codes for a protein for lysine export,
11.6 the dapD gene which codes for tetradihydrodipicolinate succinylase,
11.7 the dapE gene which codes for the succinyl diamino-pimelate desuccinylase,
11.8 the gap gene which codes for glyceraldehyde 3-phosphate dehydrogenase,
11.9 the mqo gene which codes for malate:quinone oxireductase,
11.10 the lysC gene which codes for a feed-back resistant aspartate kinase, and
11.11 the zwa1 gene which codes for the Zwa1 protein
is or are enhanced.

12. A process according to claim 10, wherein at the same time one or more of the genes chosen from the group consisting of:
12.1 the pck gene which codes for phosphoenol pyruvate carboxykinase,
12.2 the pgi gene which codes for glucose 6-phosphate isomerase,
12.3 the poxB gene which codes for pyruvate oxidase, and
12.4 the zwa2 gene which codes for the Zwa2 protein,
is or are eliminated.

13. Vector unable to replicate in Corynebacterium glutamicum for integration mutagenesis of the ccpA1 gene wherein said ccpA1 gene is a polynucleotide encoding a polypeptide having an amino acid sequence which is at least 90% identical to the amino acid sequence set out in SEQ ID NO: 2 and wherein said vector carries at least 15 successive nucleotides of the sequence set out in SEQ ID NO:1.

14. The vector of claim 13, wherein said vector is pCR2.1ccpAlint deposited in the E. coli strain TOP10F/pCR2.1ccpAlint under DSM 13617 at the Deutsche Sammlung für Mikroorganismen und Zellenkulturen [German Collection of Microorganisms and Cell Cultures].

15. A coryneform bacterium which contains the vector of claim 13 to 14.

16. The coryneform bacterium of claim 15, wherein said bacterium belongs to the species Corynebacterium glutamicum.

## Patentansprüche

1. Isoliertes coryneformes Bakterium, in dem die Expression eines Polynukleotids, das für ein Polypeptid kodiert, das eine Aminosäuresequenz aufweist, die zu mindestens 90% mit der Aminosäuresequenz gemäß SEQ ID NO. 2 identisch ist, ausgeschaltet ist, wobei das Polypeptid die Funktion eines Katabolit-Kontroll-Proteins aufweist.

2. Bakterium nach Anspruch 1, bei dem es sich um ein Bakterium der Art Corynebacterium glutamicum handelt.

3. Bakterium nach Anspruch 1, bei dem das Polypeptid die in SEQ ID NO. 2 dargestellte Aminosäuresequenz aufweist.

4. Bakterium nach Anspruch 1, bei dem das Polynukleotid die Nukleotidsequenz der Nukleotide 225 bis 1388 von SEQ ID NO. 1 aufweist.

5. Bakterium nach Anspruch 4, bei dem das Polynukleotid die Nukleotidsequenz von SEQ ID NO. 1 aufweist.

6. Bakterium nach Anspruch 1 oder 2 mit der Fähigkeit, L-Lysin in seinen Zellen oder in einem Medium anzureichern.

7. Verfahren zur Herstellung von L-Lysin, bei dem man die L-Lysin produzierenden Bakterien gemäß einem der Ansprüche 1 bis 6 fermentiert.

8. Verfahren zur Herstellung von L-Lysin, bei dem man
8.1 die Bakterien gemäß den Ansprüchen 1 bis 6 fermentiert,
8.2 das L-Lysin im Medium oder in den Zellen der Bakterien anreichert und
8.3 das L-Lysin isoliert.

9. Verfahren nach Anspruch 7 oder 8, bei dem man Bakterien einsetzt, in denen man zusätzlich weitere Gene des Biosyntheseweges von L-Lysin verstärkt.

10. Verfahren nach einem oder mehreren der Ansprüche 7 bis 9, bei dem man Bakterien einsetzt, in denen die Stoffwechselwege ausgeschaltet sind, die die Bildung von L-Lysin verringern.

11. Verfahren nach Anspruch 9, bei dem man Bakterien fermentiert, in denen man gleichzeitig eines oder mehrere der Gene, ausgewählt aus der Gruppe
11.1 das für die Dihydrodipicolinat-Synthase kodierende Gen dapA,
11.2 das für die Enolase kodierende Gen eno,
11.3 das für das zwf-Genprodukt kodierende Gen zwf,
11.4 das für die Pyruvat-Carboxylase kodierende Gen pyc,
11.5 das für den Lysin-Export kodierende Gen lysE,
11.6 das für die Tetradihydrodipicolinat-Succinylase kodierende Gen dapD,
11.7 das für die Succinyldiaminopimelat-Desuccinylase kodierende Gen dapE.,
11.8 das für die Glyceraldehyd-3-phosphat-Dehydrogenase kodierende Gen gap,
11.9 das für die Malat:Chinon-Oxidoreduktase kodierende Gen mqo,
11.10 das für eine feedbackresistente Aspartkinase kodierende Gen lysC und
11.11 das für das Zwa1-Protein kodierende Gen zwa1,
verstärkt.

12. Verfahren nach Anspruch 10, bei dem man gleichzeitig eines oder mehrere der Gene, ausgewählt aus der Gruppe
12.1 das für die Phosphoenolpyruvat-Carboxykinase kodierende Gen pck,
12.2 das für die Glucose-6-phosphat-Isomerase kodierende Gen pgi,
12.3 das für die Pyruvat-Oxidase kodierende Gen poxB und
12.4 das für das Zwa2-Protein kodierende Gen zwa2,
ausschaltet.

13. In Corynebacterium glutamicum nicht replikativer Vektor für die Integrationsmutagenese des Gens ccpA1, wobei es sich bei dem Gen ccpA1 um ein Polynukleotid handelt, das für ein Polypeptid kodiert, das eine Aminosäuresequenz aufweist, die zu mindestens 90% mit der in SEQ ID NO. 2 dargestellten Aminosäuresequenz identisch ist, wobei der Vektor mindestens 15 aufeinanderfolgende Nukleotide der in SEQ ID NO. 1 dargestellten Sequenz trägt.

14. Vektor nach Anspruch 13, bei dem es sich um pCR2.1ccpA1int, hinterlegt in dem E. coli-Stamm Top10F/pCR2.1ccpA1int unter der Nummer DSM 13617 bei der Deutschen Sammlung für Mikrorganismen und Zellenkulturen, handelt.

15. Coryneformes Bakterium, enthaltend den Vektor gemäß Anspruch 13 bis 14.

16. Coryneformes Bakterium nach Anspruch 15, das zu der Art Corynebacterium glutamicum gehört.

## Revendications

1. Bactérie coryneforme isolée, dans laquelle l'expression d'un polynucléotide codant pour un polypeptide ayant une séquence d'acides aminés qui est identique au moins à 90% à la séquence d'acides aminés représentée dans la SEQ ID n° 2 est éliminée, dans laquelle le polypeptide ayant la fonction d'une protéine de contrôle de catabolites.

2. Bactérie selon la revendication 1, ladite bactérie étant une bactérie de l'espèce Corynebacterium glutamicum.

3. Bactérie selon la revendication 1, dans laquelle ledit polypeptide a la séquence d'acides aminés représentée dans la SEQ ID n° 2.

4. Bactérie selon la revendication 1, dans laquelle ledit polynucléotide a la séquence nucléotidique couvrant les nucléotides 225 à 1388 de la SEQ ID n° 1.

5. Bactérie selon la revendication 4, dans laquelle ledit polynucléotide a la séquence nucléotidique de la SEQ ID n° 1.

6. Bactérie selon la revendication 1 ou 2, ladite bactérie ayant la capacité d'accumuler la L-lysine dans ses cellules ou dans un milieu.

7. Procédé de préparation de L-lysine comprenant:
- la fermentation des bactéries selon l'une des revendications 1 à 6, qui produisent de la L-lysine.

8. Procédé de préparation de L-lysine comprenant:
8.1 la fermentation des bactéries selon les revendications 1 à 6,
8.2 la concentration de la L-lysine dans le milieu ou dans les cellules bactériennes, et
8.3 l'isolement de la L-lysine.

9. Procédé selon la revendication 7 ou 8, dans lequel on utilise des bactéries dans lesquelles on renforce en outre d'autres gènes de la voie de biosynthèse de la L-lysine.

10. Procédé selon l'une ou plusieurs des revendications 7 à 9, dans lequel on utilise des bactéries dans lesquelles on a éliminé les voies métaboliques qui réduisent la formation de L-lysine.

11. Procédé selon la revendication 9, dans lequel on fait fermenter des bactéries dans lesquelles on a renforcé, en même temps, un ou plusieurs gènes choisis dans le groupe constitué:
11.1 du gène dapA qui code pour la dihydrodipicolinate-synthase,
11.2 du gène eno qui code pour l'énolase,
11.3 du gène zwf qui code pour le produit du gène zwf,
11.4 du gène pyc qui code pour la pyruvate-carboxylase,
11.5 du gène lysE qui code pour une protéine d'export de la lysine,
11.6 du gène dapD qui code pour la tétradihydro-dipicolinate-succinylase,
11.7 du gène dapE qui code pour la succinyl-diamino-pimélate désuccinylase,
11.8 du gène gap qui code pour la glycéraldéhyde-3-phosphate déshydrogénase,
11.9 du gène mqo qui code pour la malate:quinone-oxydoréductase,
11.10 du gène lysC qui code pour une aspartate-kinase résistant à la rétroaction, et
11.11 du gène zwa1 qui code pour la protéine Zwa1.

12. Procédé selon la revendication 10, dans lequel on a éliminé, en même temps, un ou plusieurs gènes choisis dans le groupe constitué :
12.1 du gène pck qui code pour la phosphoénol-pyruvate-carboxykinase,
12.2 du gène pgi qui code pour la glucose-6-phosphate isomérase,
12.3 du gène poxB qui code pour la pyruvate-oxydase, et
12.4 du gène zwa2 qui code pour la protéine Zwa2.

13. Vecteur incapable de se répliquer dans l'espèce Corynebacterium glutamicum, en vue d'effectuer une mutagenèse par intégration du gène ccpAl, dans lequel ledit gène ccpA1 est un polynucléotide codant pour un polypeptide ayant une séquence d'acides aminés qui est identique au moins à 90% à la séquence d'acides aminés représentée dans la SEQ ID n° 2, et dans lequel ledit vecteur porte au moins 15 nucléotides successifs de la séquence représentée dans la SEQ ID n° 1.

14. Vecteur selon la revendication 13, dans lequel ledit vecteur est le vecteur pCR2.1ccpAlint déposé dans la souche TOP10F/pCR2.1ccpA1int d'E. coli sous la référence DSM 13617 auprès de la Deutsche Sammlung für Mikroorganismen und Zellenkulturen (collection allemande de microorganismes et de cultures cellulaires).

15. Bactérie coryneforme, qui contient le vecteur selon les revendications 13 et 14.

16. Bactérie coryneforme selon la revendication 15, ladite bactérie appartenant à l'espèce Corynebacterium glutamicum.
